(19) Europäisches Patentamt
European Patent Office
Office européen des brevets

(11) Veröffentlichungsnummer: **0 019 130**
**B1**

(12) **EUROPÄISCHE PATENTSCHRIFT**

(45) Veröffentlichungstag der Patentschrift: **07.07.82**

(21) Anmeldenummer: **80102289.8**

(22) Anmeldetag: **28.04.80**

(51) Int. Cl.³: **A 61 K 31/41,** A 01 N 43/64 // C07D249/08, C07C49/16, C07C49/167, C07C49/245, C07C143/68

(54) **Antimykotische Mittel und Verfahren zur Herstellung dieser Mittel.**

(30) Priorität: **10.05.79 DE 2918896**

(43) Veröffentlichungstag der Anmeldung: **26.11.80 Patentblatt 80/24**

(45) Bekanntmachung des Hinweises auf die Patenterteilung: **07.07.82 Patentblatt 82/27**

(84) Benannte Vertragsstaaten: **AT BE CH DE FR GB IT LI NL SE**

(56) Entgegenhaltungen:
**EP-A-0 004 303**
**EP-A-0 006 538**
**EP-A-0 009 707**
**DE-A-2 632 603**
**DE-A-2 811 916**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Krämer, Wolfgang, Dr., Am Eckbusch 39/162, D-5600 Wuppertal-1 (DE)**
Erfinder: **Büchel, Karl Heinz, Dr., Bergerheide 62, D-5600 Wuppertal-1 (DE)**
Erfinder: **Stetter, Jörg, Dr., Gellertweg 4, D-5600 Wuppertal-1 (DE)**
Erfinder: **Plempel, Manfred, Dr., Pahlkestrasse 5, D-5600 Wuppertal-1 (DE)**
Erfinder: **Haller, Ingo, Dr., Viktoriastrasse 99, D-5600 Wuppertal-1 (DE)**

**EP 0 019 130 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Antimykotische Mittel und Verfahren zur Herstellung dieser Mittel

Die Erfindung betrifft antimykotische Mittel, enthaltend neue fluorierte 1-Triazolyl-butan-Derivate sowie Verfahren zur Herstellung dieser Mittel.

Es ist bereits bekannt geworden, dass chlorierte und bromierte 1-Triazolyl-butan-Derivate gute fungizide Eigenschaften aufweisen (vgl. DE-OS-26 32 603). Ihre antimykotische Wirksamkeit ist jedoch, insbesondere in-vivo gegen Candida, nicht befriedigend.

Es wurde gefunden, dass die neuen fluorierten 1-Triazolyl-butan-Derivate der allgemeinen Formel I

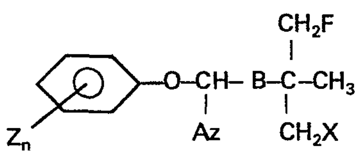 (I)

in welcher

Az für 1,2,4-Triazol-1-yl oder 1,2,4-Triazol-4-yl steht,

B für die Ketogruppe oder die CH(OH)-Gruppierung steht,

X für Wasserstoff oder Fluor steht,

Z für Halogen, Alkyl mit 1–4 C-Atomen, Nitro, Cyano, Alkoxycarbonyl mit 1–4 C-Atomen im Alkylteil oder für gegebenenfalls durch Halogen substituiertes Phenyl steht und

n für 0, 1,2 oder 3 steht, und deren physiologisch verträglichen Saureadditions-Salze und Metallsalz-Komplexe gute antimykotische Eigenschaften aufweisen.

Diejenigen Verbindungen der allgegemeinen Formel I, in welchen B für die CH(OH)-Gruppierung steht, besitzen zwei asymmetrsiche Kohlenstoffatome; sie können deshalb in den beiden geometrischen Isomeren (threo- und erythro-Form) vorliegen, die in unterschiedlichen Mengenverhältnissen anfallen können. In beiden Fällen liegen sie als optische Isomeren vor. Sämtliche Isomeren werden erfindungsgemäss beansprucht.

Überraschenderweise zeigen die erfindungsgemässen fluorierten 1-Triazolyl-butan-Derivate der allgemeinen Formel I eine bessere antimykotische Wirksamkeit, insbesondere in-vivo gegen Candida, als die bekannten chlorierten und bromierten 1-Triazolyl-butan-Derivate, die chemisch die naheliegendsten Verbindungen sind. Die erfindungsgemässen Mittel stellen somit eine Bereicherung der Pharmazie dar.

Bevorzugt sind diejenigen erfindungsgemässen Mittel, die fluorierte 1-Triazolyl-butan-Derivate enthalten, in denen Z für Fluor, Chlor, Brom, Jod, Methyl, Ethyl, Nitro, Cyano, Methoxycarbonyl, Ethoxycarbonyl, Phenyl oder Chlorphenyl steht, der Index n für 0, 1 oder 2 steht und Az, B sowie X die in der Erfindungsdefinition angegebene Bedeutung haben.

Im einzelnen seien ausser den bei den Herstellungsbeispielen genannten Verbindungen die folgenden Verbindungen der allgemeinen Formel I genannt:

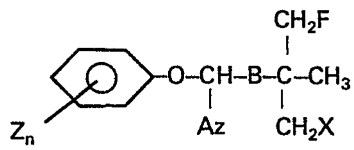 (I)

| $Z_n$ | X | B | Az |
|---|---|---|---|
| – | H | CO | 1,2,4-Triazol-1-yl |
| 2–F | H | CO | 1,2,4-Triazol-1-yl |
| 3–F | H | CO | 1,2,4-Triazol-1-yl |
| 4–F | H | CO | 1,2,4-Triazol-1-yl |
| 2–Cl | H | CO | 1,2,4-Triazol-1-yl |
| 3–Cl | H | CO | 1,2,4-Triazol-1-yl |
| 2–Br | H | CO | 1,2,4-Triazol-1-yl |
| 3–Br | H | CO | 1,2,4-Triazol-1-yl |
| 2–$CH_3$ | H | CO | 1,2,4-Triazol-1-yl |
| 4–$CH_3$ | H | CO | 1,2,4-Triazol-1-yl |
| 2– 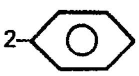 | H | CO | 1,2,4-Triazol-1-yl |
| 4– 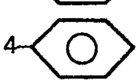 | H | CO | 1,2,4-Triazol-1-yl |
| 4– 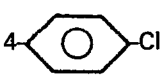 | H | CO | 1,2,4-Triazol-1-yl |
| 2–$NO_2$ | H | CO | 1,2,4-Triazol-1-yl |
| 4–CN | H | CO | 1,2,4-Triazol-1-yl |
| 4–$COOCH_3$ | H | CO | 1,2,4-Triazol-1-yl |
| 4–$COOC_2H_5$ | H | CO | 1,2,4-Triazol-1-yl |
| 4–J | H | CO | 1,2,4-Triazol-1-yl |
| 4–Cl,2–$CH_3$ | H | CO | 1,2,4-Triazol-1-yl |
| 4–$CH_3$,2–Cl | H | CO | 1,2,4-Triazol-1-yl |

| $Z_n$ | X | B | Az |
|---|---|---|---|
| – | H | CH(OH) | 1,2,4-Triazol-1-yl |
| 2–F | H | CH(OH) | 1,2,4-Triazol-1-yl |
| 3–F | H | CH(OH) | 1,2,4-Triazol-1-yl |
| 4–F | H | CH(OH) | 1,2,4-Triazol-1-yl |
| 2–Cl | H | CH(OH) | 1,2,4-Triazol-1-yl |
| 3–Cl | H | CH(OH) | 1,2,4-Triazol-1-yl |
| 2–Br | H | CH(OH) | 1,2,4-Triazol-1-yl |
| 3–Br | H | CH(OH) | 1,2,4-Triazol-1-yl |
| 2–$CH_3$ | H | CH(OH) | 1,2,4-Triazol-1-yl |
| 4–$CH_3$ | H | CH(OH) | 1,2,4-Triazol-1-yl |
| 2– 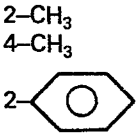 | H | CH(OH) | 1,2,4-Triazol-1-yl |
| 4– 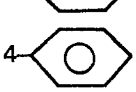 | H | CH(OH) | 1,2,4-Triazol-1-yl |
| 4– 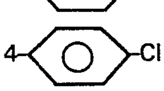 | H | CH(OH) | 1,2,4-Triazol-1-yl |
| 2–$NO_2$ | H | CH(OH) | 1,2,4-Triazol-1-yl |
| 4–CN | H | CH(OH) | 1,2,4-Triazol-1-yl |
| 4–$COOCH_3$ | H | CH(OH) | 1,2,4-Triazol-1-yl |

(Fortsetzung)

| $Z_n$ | X | B | Az |
|---|---|---|---|
| 4-$COOC_2H_5$ | H | CH(OH) | 1,2,4-Triazol-1-yl |
| 4-J | H | CH(OH) | 1,2,4-Triazol-1-yl |
| 4-Cl,2-$CH_3$ | H | CH(OH) | 1,2,4-Triazol-1-yl |
| 4-$CH_3$,2-Cl | H | CH(OH) | 1,2,4-Triazol-1-yl |

| $Z_n$ | X | B | Az |
|---|---|---|---|
| – | F | CO | 1,2,4-Triazol-1-yl |
| 2-F | F | CO | 1,2,4-Triazol-1-yl |
| 3-F | F | CO | 1,2,4-Triazol-1-yl |
| 3-Cl | F | CO | 1,2,4-Triazol-1-yl |
| 2-Br | F | CO | 1,2,4-Triazol-1-yl |
| 3-Br | F | CO | 1,2,4-Triazol-1-yl |
| 4-Br | F | CO | 1,2,4-Triazol-1-yl |
| 2-$CH_3$ | F | CO | 1,2,4-Triazol-1-yl |
| 4-$CH_3$ | F | CO | 1,2,4-Triazol-1-yl |
| 2-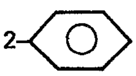 | F | CO | 1,2,4-Triazol-1-yl |
| 4-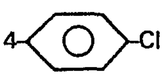 | F | CO | 1,2,4-Triazol-1-yl |
| 2-$NO_2$ | F | CO | 1,2,4-Triazol-1-yl |
| 4-$NO_2$ | F | CO | 1,2,4-Triazol-1-yl |
| 4-CN | F | CO | 1,2,4-Triazol-1-yl |
| 4-$COOCH_3$ | F | CO | 1,2,4-Triazol-1-yl |
| 4-$COOC_2H_5$ | F | CO | 1,2,4-Triazol-1-yl |
| 4-J | F | CO | 1,2,4-Triazol-1-yl |
| 4-Cl,2-$CH_3$ | F | CO | 1,2,4-Triazol-1-yl |

| $Z_n$ | X | B | Az |
|---|---|---|---|
| – | F | CH(OH) | 1,2,4-Triazol-1-yl |
| 2-F | F | CH(OH) | 1,2,4-Triazol-1-yl |
| 3-F | F | CH(OH) | 1,2,4-Triazol-1-yl |
| 3-Cl | F | CH(OH) | 1,2,4-Triazol-1-yl |
| 2-Br | F | CH(OH) | 1,2,4-Triazol-1-yl |
| 3-Br | F | CH(OH) | 1,2,4-Triazol-1-yl |
| 4-Br | F | CH(OH) | 1,2,4-Triazol-1-yl |
| 2-$CH_3$ | F | CH(OH) | 1,2,4-Triazol-1-yl |
| 4-$CH_3$ | F | CH(OH) | 1,2,4-Triazol-1-yl |
| 2-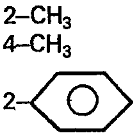 | F | CH(OH) | 1,2,4-Triazol-1-yl |
| 4-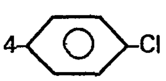 | F | CH(OH) | 1,2,4-Triazol-1-yl |
| 2-$NO_2$ | F | CH(OH) | 1,2,4-Triazol-1-yl |
| 4-$NO_2$ | F | CH(OH) | 1,2,4-Triazol-1-yl |
| 4-CN | F | CH(OH) | 1,2,4-Triazol-1-yl |
| 4-$COOCH_3$ | F | CH(OH) | 1,2,4-Triazol-1-yl |
| 4-$COOC_2H_5$ | F | CH(OH) | 1,2,4-Triazol-1-yl |
| 4-J | F | CH(OH) | 1,2,4-Triazol-1-yl |
| 4-Cl,2-$CH_3$ | F | CH(OH) | 1,2,4-Triazol-1-yl |
| 4-$CH_3$,2-Cl | F | CH(OH) | 1,2,4-Triazol-1-yl |

Die erfindungsgemässen Wirkstoffe, deren Säureadditions-Salze und Metallsalz-Komplexe sind noch nicht bekannt. Sie können jedoch gemäss einem eigenen Vorschlag hergestellt werden, indem man Halogenetherketone der allgemeinen Formel II

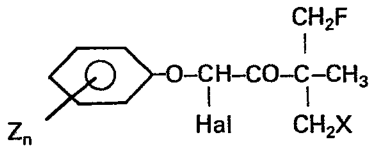 (II)

in welcher

X, Z und n die oben angegebene Bedeutung haben und

Hal für Halogen, vorzugsweise Chlor oder Brom steht, mit 1,2,4-Triazol in Gegenwart eines Säurebindemittels, wie beispielsweise Natriumcarbonat oder ein Überschuss an Triazol, und gegebenenfalls in Gegenwart eines inerten organischen Lösungsmittels, wie beispielsweise Aceton oder Acetonitril, bei Temperaturen zwischen 60 und 120 °C umsetzt und gegebenenfalls noch die erhaltenen Keto-Derivate nach bekannten Methoden reduziert, wie z.B. durch Umsetzung mit komplexen Hydriden, wie insbesondere Natriumborhydrid, gegebenenfalls in Gegenwart eines polaren organischen Lösungsmittels, wie beispielsweise Alkohole, bei Temperaturen zwischen 0 und 30 °C; oder durch Umsetzung mit Aluminiumisopropylat in Gegenwart eines inerten organischen Lösungsmittels, wie beispielsweise Isopropanol, bei Temperaturen zwischen 20 und 120 °C. Die Aufarbeitung erfolgt in üblicher Weise; gegebenenfalls wird das Salz oder ein Metallkomplex hergestellt. In manchen Fällen erweist es sich als vorteilhaft, die Verbindung der allgemeinen Formel I über ihre Salze in reiner Form zu erhalten.

Die Halogenetherketone der allgemeinen Formel II sind noch nicht bekannt. Sie können jedoch nach bekannten Verfahren (vgl. z.B. DE-OS 26 32 603) erhalten werden, indem man z.B. bekannte Phenole der allgemeinen Formel III

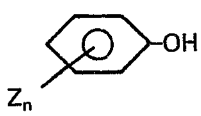 (III)

in welcher

Z und n die oben angegebene Bedeutung haben, mit einem Halogenketon der allgemeinen Formel IV

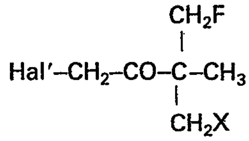 (IV)

in welcher

X die oben angegebene Bedeutung hat und

Hal' für Chlor oder Brom steht, umsetzt. Das noch verbliebene aktive Wasserstoffatom wird anschliessend in üblicher Weise gegen Halogen ausgetauscht (vgl. auch die Herstellungsbeispiele).

Die Halogenketone der allgemeinen Formel IV sind ebenfalls noch nicht bekannt. Sie können jedoch auf allgemein übliche und bekannte Weise erhalten werden, indem man Fluorderivate des

3,3-Dimethyl-butan-2-ons der allgemeinen Formel V

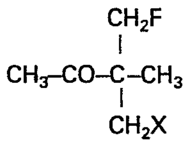

(V)

in welcher

X die oben angegebene Bedeutung hat, mit Chlor oder Brom in Gegenwart eines inerten organischen Lösungsmittels, wie beispielsweise Ether oder chlorierte Kohlenwasserstoffe, bei Raumtemperatur versetzt (vgl. auch die Herstellungsbeispiele), oder mit üblichen Chlorierungsmitteln, wie beispielsweise Sulfurylchlorid bei 20 bis 60 °C umsetzt.

Die Fluorderivate des 3,3-Dimethyl-butan-2-ons der allgemeinen Formel V sind Gegenstand einer eigenen älteren Patentanmeldung, die als DE-OS 28 43 767 am 17. April 1980 offengelegt worden ist. Man erhält die Fluorderivate des 3,3-Dimethylbutan-2-ons der allgemeinen Formel V, wenn man Sulfonsäureester der allgemeinen Formel VI

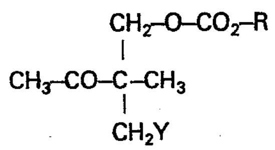

(VI)

in welcher

R für Alkyl mit 1 bis 4 Kohlenstoffatome, insbesondere Methyl, oder Aryl mit 6 bis 12 Kohlenstoffatomen, insbesondere Phenyl oder Tolyl, steht und

Y für Wasserstoff oder die Gruppe $-O-SO_2-R$ steht, mit Metallfluoriden, wie beispielsweise Natrium- und Kaliumflorid, in Gegenwart eines polaren organischen Lösungsmittels, wie beispielsweise Di-, Tri- oder Tetraäthylenglykol, bei Temperaturen zwischen 80 und 250 °C umsetzt (vgl. auch die Herstellungsbeispiele).

Die Sulfonsäureester der allgemeinen Formel VI sind teilweise bekannt [J. Org. chem. 35, 2391 (1970)]. Die noch nicht beschriebenen Verbindungen können nach literaturbekannten Verfahren aus den entsprechenden Hydroxybutanonen und Sulfochloriden in Gegenwart von Basen hergestellt werden (siehe z.B. Houben-Weyl, Methoden der Org. Chemie, Bd IX, S.388 u. 663, sowie die Angaben bei den Herstellungsbeispielen).

Zur Herstellung von Säureadditionssalzen der Verbindungen der allgemeinen Formel I kommen alle physiologisch verträglichen Säuren infrage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäure, wie z.B. p-Toluolsulfonsäure und 1,5-Naphthalindisulfonsäure.

Die Salze der Verbindung der allgemeinen Formel I können in einfacher Weise nach üblichen Salzbildungsmethoden, z.B. durch Lösen einer Verbindung der allgemeinen Formel I in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel I kommen vorzugsweise Salze von Metallen der II. bis IV.-Haupt- und der I. und II. sowie IV. bis VIII. Nebengruppe infrage, wobei Kupfer, Zink, Mangan, Magnesium, Zinn, Eisen und Nickel beispielhaft genannt seinen. Als Anionen der Salze kommen solche in Betracht, die sich von physiologischen Säuren ableiten. Hierzu gehören vorzugsweise die Halogenwasserstoffsäuren, wie z.B. die Chlorwasserstoffsäure und die Bromwasserstoffsäure, ferner Phosphorsäure, Salpetersäure und Schwefelsäure.

Die Metallsalzkomplexe der Verbindungen der allgemeinen Formel I können in einfacher Weise nach üblichen Verfahren erhalten werden, so z.B. durch Lösen des Metallsalzes in Alkohol, z.B. Ethanol, und Hinzufügen zur Verbindung der allgemeinen Formel I. Man kann Metallkomplexe in bekannter Weise, z.B. durch Abfiltrieren, isolieren und gegebenenfalls durch Umkristallisieren reinigen.

Die erfindungsgemässen Verbindungen der allgemeinen Formel I, ihre Säureadditions-Salze und Metallsalz-Komplexe weisen starke antimykotische Wirkungen auf. Sie besitzen ein sehr breites antimykotisches Wirkungsspektrum, insbesondere gegen Dermatophyten und Sprosspilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, wie Trichophyton-Arten, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sprosspilze und biphasische Pilze sowie Schimmelpilze hervorgerufen.

Als Indikationsgebiet in der Tiermedizin können beispielsweise aufgeführt werden:

Alle Dermatomykosen und Systemmykosen, insbesondere solche, die durch die obengenannten Erreger hervorgerufen werden.

Zur Erfindung gehören auch pharmazeutische Zubereitungen, die neben nicht toxischen, inerten pharmazeutisch geeigneten Trägerstoffen einen oder mehrere erfindungsgemässe Wirkstoffe enthalten oder die aus einem oder mehreren erfindungsgemässen Wirkstoffen bestehen sowie Verfahren zur Herstellung dieser Zubereitungen.

Zur Erfindung gehören schliesslich auch pharmazeutische Zubereitungen in Dosierungseinheiten. Dies bedeutet, dass die Zubereitungen in Form einzelner Teile, z.B. Tabelle, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder $^1/_2$, $^1/_3$ oder $^1/_4$ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seinen Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzukker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmitel, z.B. Agar-Agar, Calciumcarbonat und Natriumbicarbonat, (e) Lösungsverzögerer, z.B. Praffin und (f) Resorptionsbeschleuniger, z.B. quartenäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magensiumstearat und feste Polyäthylenglykole oder Gemische der unter (a) bis (i) aufgeführten Stoffe.

Den Tabletten, Dragees, Kapseln, Pillen und Granulate können mit den üblichen gegebenenfalls Opakisierungsmittel enthaltenden Überzugen und Hüllen versehen sein und auch so zusammengesetzt sein, dass sie den oder die Wirkstoffe nur oder bevorzugt in einem betimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Äthylalkohol, Propylenglykol, Suspendiermittel, z.B. äthoxylierte Isostearylalkohole, Polyoxyäthylensorbit- und Sorbitanester, mikrokristalline Cellulose, Aluminiummethahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z.B. Pfefferminzöl und Eukalyptusöl und Süssmittel, z.B. Sacharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können ausser den erfindungsgemässen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemein hat es sich sowohl in der Humanals auch in der Veterinärmedizin als vorteilhaft erwiesen, den oder die erfindungsgemässen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

Beispiel A

Antimykotische in-vitro-Wirksamkeit

Versuchsbeschreibung:

Die in-vitro-Prüfung wurde im Reihenverdünnungstest mit Keiminokula von durchschnittlich 5 × 10 Keimen /ml Substrat durchgeführt. Als Nahrmedium dienten

a) für Dermatophyten und Schimmelpilze: Sabourand's milieu d'épresive

b) für Hefen:

Isotonic Sensitest-Broth von Oxid.

Die Bebrütungstemperatur betrug 28 °C; Bebrütungszeit war 24 Stunden bei Hefen und 96 Stunden bei Dermatophyten und Schimmelpilzen.

In dieser Versuchsanordnung ergeben sich für die erfindungsgemässen Mittel wesentlich bessere MHK-Werte als für die nächstliegenden bekannten Verbindungen.

Beispiel B

In-vivo-Wirksamkeit (oral) bei Mäuse-Candidose

Versuchsbeschreibung:

Mäuse vom Typ SPF–$CF_1$ wurden intravenös mit 1 – 2 × $10^6$ logaritmisch wachsenden Candida-Zellen, die in physiologischer Kochsalzlösung suspendiert waren, infiziert. Eine Stunde vor und sieben Stunden nach der Injektion werden die Tiere mit jeweils 50 – 100 mg/kg Körpergewicht der Präparate oral behandelt.

Ergebnis:

Unbehandelte Tiere starben 3 bis 6 Tage post infectionem. Die Überlebensrate am 6. Tag post infectionem betrug bei unbehandelten Kontrolltieren etwa 5%. Die nächstliegenden bekannten Verbindungen zeigten keine Wirkung, während die erfindungsgemässen Mittel Wirkung (≥ 60% Überlebende am 6. Tag p.i.) bis sehr gute Wirkung (> 95% Überlebende am 6. Tag p.i.) zeigten.

Herstellungsbeispiele

Beispiel 1

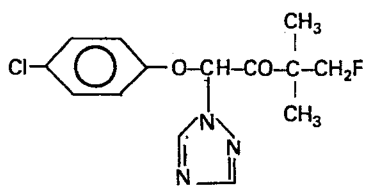

87 g (0,27 Mol) 1-Brom-1(4-chlorphenoxy)-3,3-dimethyl-4-fluor-2-butanon werden in 200 ml Aceton gelöst und zu einer siedenden Lösung von 46 g (0,66 Mol) 1,2,4-Triazol in 200 ml Aceton getropft. Nach einer Stunde Erhitzen unter Rückfluss wird das Lösungsmittel im Vakuum entfernt, der Rückstand mit Methylenchlorid aufgenommen, mehrmals mit Wasser gewaschen und die organische Phase über Natriumsulfat getrocknet. Nach Entfernen des Lösungsmittels im Vakuum kristallisiert der Rückstand aus Petrolether.

Man erhält 75 g (89% der Theorie) 1-(4-Chlorphenoxy)-3,3-dimethyl-4-fluor-1-(1,2,4-triazol-1-yl)-2-butanon vom Schmelzpunkt 60–63 °C.

Herstellung der Vorstufen

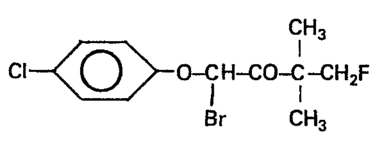

175 g (0,71 Mol( 1-(4-Chlorphenoxy)-3,3-dimethyl-4-fluor-2-butanon werden in 500 ml Methylenchlorid gelöst und bei 20–30 °C unter Rühren und Kühlen tropfenweise mit 114 g (0,71 Mol) Brom versetzt. Es wird 2 Stunden bei 20 °C nachgerührt, vorsichtig mit 200 ml Wasser versetzt, die Methylenchloridphase mehrmals mit Eiswasser gewaschen und über Natriumsulfat getrocknet. Nach Abdestillieren des Lösungsmittels im Vakuum wird der Rückstand aus Cyclohexan umkristallisiert. Man erhält 180 g (78% der Theorie) 1-Brom-1-(4-chlorphenoxy)-3,3-dimethyl-4-fluor-2-butanon vom Schmelzpunkt 73-75 °C.

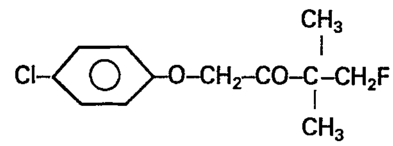

Zu einer gerührten Mischung aus 102 g (0,79 Mol) p-Chlorphenol und 110 g (0,79 Mol) gepulvertem Kaliumcarbonat in 500 ml Aceton werden unter Kühlen bei 20–30 °C 157 g (0,79 Mol) 1-Brom-3,3-dimethyl-4-fluor-2-butanon zugetropft. Es wird 2 Stunden bei 20 °C nachgerührt, das anorganische Salz abfiltriert und das Filtrat eingeengt. Der Rückstand wird im Hochvakuum destilliert. Man erhält 175 g (90% der Theorie) 1-(4-Chlorphenoxy)-3,3-dimethyl-4-fluor-2-butanon vom Siedepunkt 112–119 °C/0,05 mm Hg-Säule.

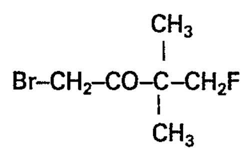

In eine Mischung aus 354 g (3 Mol) 3,3-Dimethyl-4-fluor-2-butanon und 2000 ml Ether werden bei 20–30 °C unter Kühlen und Rühren 480 g Brom langsam eingetropft. Die gelbliche Lösung wird noch 1 Stunde bei 20 °C nachgerührt und anschliessend vorsichtig mit 500 ml Wasser versetzt. Die Etherphase wird abgetrennt, mehrmals mit Wasser gewaschen und über Natriumsulfat getrocknet. Nach Abdestillieren des Lösungsmittels wird der Rückstand im Wasserstrahlvakuum destilliert. Man erhält 472 g (80% der Theorie) 1-Brom-3,3-dimethyl-4-fluor-2-butanon vom Siedepunkt 80–90 °C/11 mm Hg-Säule.

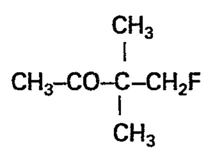

Zu der in einem Dreihals-Rührkolben mit absteigendem Kühler befindlichen Suspension von 23,2 g (0,4 Mol) trockenem Kaliumfluorid in 400 ml dest. Teträthylenglykol werden bei 160 °C und 20 mbar 38,8 g (0,2 Mol) 2,2-Dimethyl-2-oxobutyl-methansulfonat im Verlauf von 2 Stunden zugetropft und 2 weitere Stunden nachgerührt. An einem absteigenden Kondensator und in einer nachgeschalteten Tiefkühlfalle wird das herausdestillierte Reaktionsprodukt kondensiert und gesammelt. Man erhält 20,9 g (89% der Theorie) 3,3-Dimethyl-4-fluor-2-butanon, vom Siedepunkt 130–134 °C.

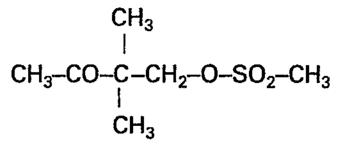

232 g (2 Mol) 3,3-Dimethyl-4-hydroxy-2-butanon (z. Herstellung vgl. Beilstein H1 E III 3239, IV 4030 und Bull.Soc.Chim.France 1964, 2849) werden in 700 ml absolutem Pyridin bei 0 bis 5 °C mit 229 g (2 Mol) Methansulfochlorid umgesetzt. Nach 12 Stunden Stehen bei 20 °C wird mit Methylenchlorid verdünnt und mit Eiswasser ausgeschüttelt. Die organische Phase wird getrocknet, im Vakuum vom Lösungsmittel befreit und über eine Kolonne fraktioniert. Man erhält 332 g (86% der Theorie) 2,2-Dimethyl-3-oxo-butyl-methansulfonat vom Siedepunkt 106–120 °C/0,12 mm Hg-Säule.

Beispiel 2

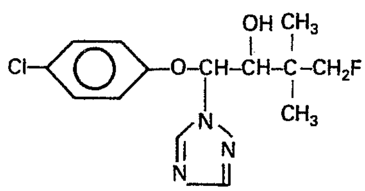

55 g (0,176 Mol) 1-(4-Chlorphenoxy)-4-fluor-3,3-dimethyl-1-(1,2,4-triazol-1-yl)-2-butanon (Beispiel 1) werden in 250 ml Methanol gelöst und portionsweise mit 3 g (0,08 Mol) Natriumborhydrid versetzt. Man lässt die Reaktionslösung 1 Stunde nachrühren und stellt sie dann mit konzentrierter Salzsäure auf einen pH-Wert von 3 ein. Nach Abdestillieren des Lösungsmittels im Vakuum wird der Rückstand mit Wasser versetzt und mit Methylenchlorid ausgeschüttelt. Die vereinigten organischen Phasen werden über Natriumsulfat getrocknet und im Vakuum eingeengt. Der Rückstand kristallisiert aus Petrolether. Man erhält 40 g (72% der Theorie) 1-(4-Chlorphenoxy)-4-fluor-3,3-dimethyl-1- (1,2,4-triazol-1-yl)-2-butanol vom Schmelzpunkt 103–112 °C.

Beispiel 3

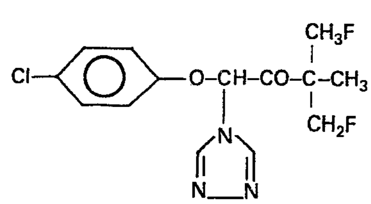

und

Beispiel 4

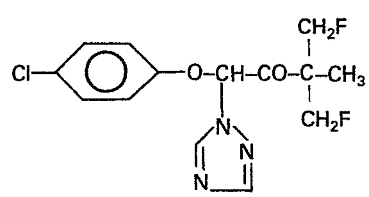

82 g (0,286 Mol) 3,3-Bisfluormethyl-1-brom-1-(4-chlorphenoxy)-butan-2-on und 41,5 g (0,588 Mol) Triazol werden in 600 ml Acetonitril vorgelegt, 5 Stunden bei 50 °C erhitzt, das Lösungsmittel im Wasserstrahlvakuum abgetragen, der Rückstand in einem Liter Methylenchlorid aufgenommen und zweimal mit je 1000 ml Wasser gewaschen, die organische Phase über Natriumsulfat getrocknet und das Lösungsmittel abdestilliert. Der Rückstand wird in 500 ml Diisopropylether aufgenommen und der Niederschlag abgesaugt. Man erhält 6 g 3,3-Bisfluormethyl-1-(4-chlorphenoxy)-1-(1,2,4-triazol-4-yl)-butan-2-on (Beispiel 3) vom Schmelzpunkt 119–122 °C. Die Mutterlauge wird destilliert. Man erhält 32,1 g (40% der Theorie) 3,3-Bisfluormethyl-1-(4-chlorphenoxy)-1-(1,2,4-triazol-1-yl)-butan-2-on (Beispiel 4) vom Siedepunkt 160–166 °C/0,2 mm HG-Säule.

Herstellung der Vorstufen

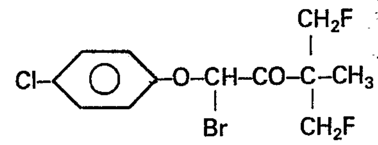

Entsprechend Beispiel 1 durch Umsetzung von 3,3-Bisfluormethyl-1-(4-chlorphenoxy)-butan-2-on mit Brom.

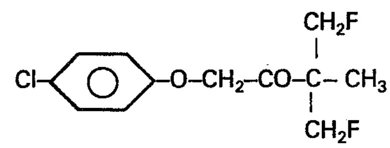

Entsprechend Beispiel 1 durch Umsetzung von p-Chlorphenol mit 3,3-Bisfluormethyl-1-brom-butan-2-on.

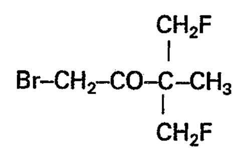

Entsprechend Beispiel 1 durch Umsetzung von 3,3-Bisfluormethyl-butan-2-on mit Brom.

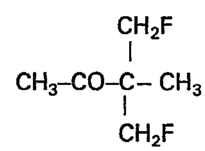

In einem Dreihalskolben mit Rührer, Tropftrichter und Liebigkühler mit gekühlter Vorlage werden 400 ml Tetraethylenglykol und 46,4 g Kaliumfluorid (0,8 Mol) vorgelegt und auf 170 °C aufgeheizt. Man legt an den Vorstoss des Liebigkühlers ein Wasserstrahlvakuum (Druck ca. 20 bis 30 mbar) an. Dann werden während 45 Minuten 57,6 g (0,2 Mol) 2-Acetyl-2-methyl-propan-1,3-diol-bismethansulfat, gelöst in 100 ml Tetraäthylenglykol, zugetropft. Das entstehende 3,3-Bisfluormethyl-butan-2-on wird während der Reaktion in die gekühlte Vorlage abdestilliert. Nach dem Zutropfen wird noch während 1 Stunde bei 175 °C weiter destilliert.

Das aufgefangene Destillat wird anschliessend redestilliert. Man erhält 14 g (ca. 51,5% der Theorie) 3,3-Bisfluormethyl-butan-2-on vom Siedepunkt 43–46 °C/12 mm HG-Säule.

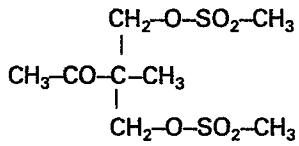

66 g (0,5 Mol) 3-Oxa-2,2-bis-(hydroxymethyl)-butan (zur Herstellung vgl. Beilstein H1, E III 3306, IV 4132 und J.Chem.Soc., London, 1932, 2671) werden in 300 ml 1,2-Dichlorethan gelöst, 114,5 g (1 Mol) Methansulfonsäurechlorid zugetropft und bei 0 bis 5 °C 158 g (2 Mol) Pyridin zugetropft. Man lässt 15 Stunden bei Raumtemperatur nachrühren und gibt dann den Ansatz auf 600 ml Eiswasser und 100 ml konz. Salzsäure. Dabei fällt ein Feststoff aus, der abgesagut wird. Die wässrige Phase wird mit 1000 ml Methylenchlorid extrahiert; in der Methylenchloridphase wird der Feststoff gelöst, die organische Phase über Natriumsulfat getrocknet, das Lösungsmittel im Wasserstrahlvakuum abdestilliert und der Rückstand in 200 ml Ether suspendiert. Der Rückstand wird abgesaugt und mit 100 ml Ether gewaschen. Man erhält 100 g (ca. 70% der Theorie) 2-Acetyl-2-methyl-propan-1,3-diol-bis-methansulfonat vom Schmelzpunkt 105–108 °C.

In entsprechender Weise werden die nachfolgenden Verbindungen der allgemeinen Formel I

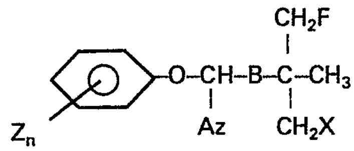 (I)

erhalten:

| Bsp. Nr. | $Z_n$ | B | X | 1,2,4-Triazol-1-yl oder -4-yl | Schmelzpunkt (°C) bzw. Siedepunkt (°C) /mm-Hg-Säule |
|---|---|---|---|---|---|
| 5 | 4–Br | CO | H | 1yl | 131(xHCl) |
| 6 | 2,4–$Cl_2$ | CO | H | 1-yl | 213(Zers.) (xl/2 NDS) |
| 7 | 4–Cl | CO | H | 1-yl | 260-65(xl/2NDS) |
| 8 | 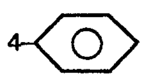 | CO | F | 1-yl | 110(xl/2 NDS) |
| 9 | 4–F | CO | F | 1-yl | 144-46/0,2 |
| 10 | 2,4–$Cl_2$ | CO | H | 4-yl | 139 |
| 11 | 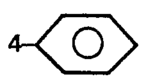 | CO | H | 4-yl | 194 |
| 12 | 4–F | CO | F | 4-yl | 108 |
| 13 | 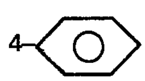 | CO | F | 4-yl | 150 |
| 14 | 2,4–$Cl_2$ | CH(OH) | H | 1-yl | 142-52 |
| 15 | 4–Br | CH(OH) | H | 1-yl | 117-40 |
| 16 | 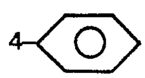 | CH(OH) | F | 1-yl | 120 |
| 17 | 4–Cl | CH(OH) | F | 1-yl | 98 |
| 18 | 4–F | CH(OH) | F | 1-yl | 95–97 |
| 19 | 2–Cl | CO | F | 1-yl | 157–60/0,3 |
| 20 | 2,4–$Cl_2$ | CO | F | 1-yl | 167/0,3 |
| 21 | 2–Cl,4–$CH_3$ | CO | F | 1-yl | 162/0,3 |
| 22 | 2–Cl | CO | F | 4-yl | 122–26 |
| 23 | 2,4–$Cl_2$ | CO | F | 4-yl | 134–40 |
| 24 | 2–Cl | CH(OH) | F | 1-yl | 96–102 |
| 25 | 2,4–$Cl_2$ | CH(OH) | F | 1-yl | 90–95 |
| 26 | 4–Cl | CH(OH) | F | 1-yl | 124 (A-Form)$^{+)}$ |
| 27 | 4–F | CH(OH) | F | 1-yl | 89–91 (A-Form) |
| 28 | 2,4–$Cl_2$ | CH(OH) | F | 1-yl | 91–93 (A-Form) |

| Bsp. Nr. | $Z_n$ | B | X | 1,2,4-Triazol-1-yl oder -4-yl | Schmelzpunkt (°C) bzw. Siedepunkt (°C)/mm-Hg-Säule |
|---|---|---|---|---|---|
| 29 | 4-Br | CO | F | 1-yl | 167–70/0,1 |
| 30 | 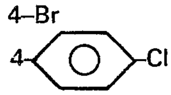 | CO | F | 1-yl | 80–94 |
| 31 | 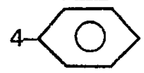 | CO | F | 1-yl | 75–80 |
| 32 | 3-Cl | CO | F | 1-yl | 169–73/0,5 |
| 33 | 4-Br | CH(OH) | F | 1-yl | 140–43 |
| 34 | 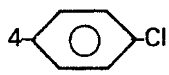 | CH(OH) | F | 1-yl | 165–68 |
| 35 | 3-Cl | CH(OH) | F | 1-yl | 98–104 |
| 36 | 4Cl, 2-$CH_3$ | CH(OH) | F | 1-yl | 108 |
| 37 | 4-$CH_3$ | CH(OH) | F | 1-yl | 109–11 |
| 38 | 4-$NO_2$ | CO | F | 1-yl | 84–86 |
| 39 | 2-F | CH(OH) | H | 1-yl | 175(xHCl) |
| 40 | 4-$CH_3$ | CO | F | 1-yl | 150–55/0,1 |
| 41 | 4-$COOCH_3$ | CO | F | 1-yl | Öl |
| 42 | 4-$COOC_2H_5$ | CO | F | 1-yl | 178–80/0,2 |
| 43 | 4-CN | CO | F | 1-yl | Öl |
| 44 | 3-Br | CO | F | 1-yl | 160–63/0,06 |
| 45 | 2-Br | CO | F | 1-yl | 162–65/0,1 |
| 46 | 3-Br | CH(OH) | F | 1-yl | 113 |
| 47 | 3,4-$Cl_2$ | CO | H | 1-yl | 60–63 |

NDS = 1,5-Naphthalindisulfonsäure

+) A- und B-Form: jeweils eine der beiden möglichen geometrischen Isomeren

**Patentansprüche**

1. Antimykotisches Mittel, gekennzeichnet durch einen Gehalt an mindestens einem fluorierten 1-Triazolyl-butan-derivat der allgemeinen Formel I

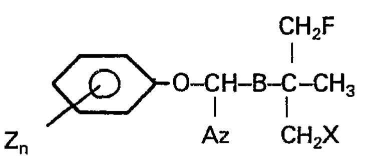

(I)

in welcher

Az für 1,2,4-Triazol-1-yl oder 1,2,4-Triazol-4-yl steht,

B für die Ketogruppe oder die CH(OH)-Gruppierung steht,

X für Wasserstoff oder Fluor steht,

Z für Halogen, Alkyl mit 1–4 C-Athomen, Nitro, Cyano, Alkoxycarbonyl mit 1–4 C-Atomen im Alkylteil oder für gegebenenfalls durch Halogen substituiertes Phenyl steht, und

n für 0, 1, 2 oder 3 steht, und/oder deren physiologisch verträglichen Säureadditions-Salze und/oder Metallkomplexe.

2. Antimykotisches Mittel gemäss Anspruch 1, gekennzeichnet durch einen Gehalt an mindestens einem fluorierten 1-Triazolderivat der allgemeinen Formel I in Anspruch 1, in welcher

Z für Halogen, Methyl, Ethyl, Nitro, Cyano, Methoxycarbonyl, Ethoxycarbonyl, Phenyl oder Chlorphenyl steht,

n für 0, 1 oder 2 steht und Az, B und X die oben angegebene Bedeutung haben.

3. Antimykotisches Mittel gemäss Anspruch 1, gekennzeichnet durch einen Gehalt an 1-(4-Chlorphenoxy)-3,3-dimethyl-4-fluor-1-)1,2,4-triazol-1-yl)-2-butanon.

4. Antimykotisches Mittel gemäss Anspruch 1, gekennzeichnet durch einen Gehalt an 1-(4-Chlorphenoxy)-3,3-dimethyl-4-fluor-1-(1,2,4-triazol-1-yl)-2-butanol.

5. Antimykotisches Mittel gemäss Anspruch 1, gekennzeichnet durch einen Gehalt an 1-(4-Chlorphenoxy)-3,3-di-(fluormethyl-1-(1,2,4-triazol-1-yl)-2-butanon.

6. Antimykotisches Mittel gemäss Anspruch 1, gekennzeichnet durch einen Gehalt an 1-(4-Chlorphenoxy)-3,3-di-(fluormethyl)-1-(1,2,4-triazol-1-yl)-2-butanol.

7. Antimykotisches Mittel gemäss Anspruch 1, gekennzeichnet durch einen Gehalt an 1-(4-Fluorphenoxy)-3,3-di-(fluormethyl)-1-(1,2,4-triazol-1-yl)-2-butanol.

8. Verfahren zur Herstellung eines antimykotischen Mittels, dadurch gekennzeichnet, dass man fluorierte 1-Triazolylbutan-derivate gemäss der allgemeinen Formel I in Anspruch 1 mit inerten, nichttoxischen, pharmazeutisch geeigneten Trägerstoffen vermischt.

**Revendications**

1. Agent antimycotique, caractérisé en ce qu'il contient au moins un dérivé fluoré du 1-triazolyl-butane, de formule générale I

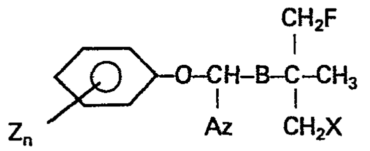

(I)

dans laquelle

Az représente un groupe 1,2,4-triazole-1-yle ou 1,2,4-triazole-4-yle,

B représente le groupe céto ou le groupement CH(OH)-,

X représente l'hydrogène ou le fluor,

Z représente un halogène, un groupe alkyle en $C_1$–$C_4$, nitro, cyano, alcoxycarbonyle contenant de 1 à 4 atomes de carbone dans la partie alkyle ou bien un groupe phényle éventuellement substitué par un halogène, et

n est égal à 0, 1, 2 ou 3, et/ou leurs sels formés par addition avec des acides acceptables pour l'usage pharmaceutique et/ou complexes métalliques.

2. Agent antimycotique selon la revendication 1, caractérisé en ce qu'il contient au moins un dérivé fluoré de 1-triazole de formule générale I de la revendication 1 dans laquelle

Z représente un halogène, un groupe méthyle, éthyle, nitro, cyano, méthoxycarbonyle, éthoxycarbonyle, phényle ou chlorophényle,

n est égal à 0, 1 ou 2,

Az, B et X ont les significations indiquées ci-dessus.

3. Agent antimycotique selon la revendication 1, caractérisé en ce qu'il contient de la 1-(4-chlorophénoxy)-3,3-diméthyl-4-fluoro-1-(1,2,4-triazole-1-yl)-2-butanone.

4. Agent antimycotique selon la revendication 1, caractérisé en ce qu'il contient du 1-(4-chlorophénoxy)-3,3-diméthyl-4-fluoro-1-(1,2,4-triazole-1-yl)-2-butanol.

5. Agent antimycotique selon la revendication 1, caractérisé en ce qu'il contient de la 1-(4-chlorophénoxy)-3,3-di-(fluorométhyl)-1-(1,2,4-triazole-1-yl)-2-butanone.

6. Agent antimycotique selon la revendication 1, caractérisé en ce qu'il contient du 1-(4-chlorophénoxy)-3,3-di-(fluorométhyl)-1-(1,2,4-tirazole-1-yl)-2-butanol.

7. Agent antimycotique selon la revendication 1, caractérisé en ce qu'il contient du 1-(4-fluorophénoxy)-3,3-di-(fluorométhyl)-1-(1,2,4-triazole-1-yl)-2-butanol.

8. Procédé de préparation d'un agent antimycotique, caractérisé en ce que l'on mélange des dérivés fluorés du 1-triazolyl-butane de formule générale I selon la revendication 1 avec des véhicules inertes, non toxiques, appropriés à l'usage pharmaceutique.

**Claims**

1. Antimycotic agent, characterised in that it contains at least one fluorinated 1-triazolyl-butane derivative of the general formula I

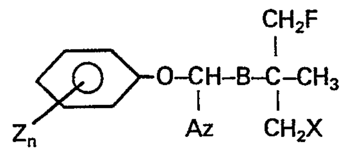

(I)

in which

Az represents 1,2,4-triazol-1-yl or 1,2,4-triazol-4-yl,

B represents the keto group or the CH(OH)-grouping,

X represents hydrogen or fluorine,

Z represents halogen, alkyl with 1–4 carbon atoms, nitro, cyano, alkoxycarbonyl with 1–4 carbon atoms in the alkyl part or phenyl which is optionally substituted by halogen, and

n represents 0, 1, 2 or 3, and/or physiologically acceptable acid addition salts and/or metal complexes thereof.

2. Antimycotic agent according to Claim 1, characterised in that it contains at least one fluorinated 1-triazolyl-butane derivative of the general formula I in Claim 1, in which Z represents halogen, methyl, ethyl, nitro, cyano, methoxycarbonyl, ethoxycarbonyl, phenyl or chlorophenyl, n represents 0, 1 or 2 and Az, B and X have the meaning indicated above.

3. Antimycotic agent according to Claim 1, characterised in that it contains 1-(4-chlorophenoxy)-3,3-dimethyl-4-fluoro-1-(1,2,4-triazol-1-yl)-2-butanone.

4. Antimycotic agent according to Claim 1, characterised in that it contains 1-(4-chlorophenoxy)-3,3-dimethyl-4-fluoro-1-(1,2,4-triazol-1-yl)-2-butanol.

5. Antimycotic agent according to Claim 1, characterised in that it contains 1-(4-chlorphenoxy)-3,3-di-(fluoromethyl)-1-(1,2,4-triazol-1-yl)-2-butanone.

6. Antimycotic agent according to Claim 1, characterised in that it contains 1-(4-chlorophenoxy)-3,3-di-(fluoromethyl)-1-(1,2,4-tirazol-1-yl)-2-butanol.

7. Antimycotic agent according to Claim 1, characterised in that it contains 1-(4-fluorophenoxy)-3,3-di-(fluoromethyl)-1-(1,2,4-triazol-1-yl)-2-butanol.

8. Process for the preparation of an antimycotic agent, characterised in that fluorinated 1-triazolyl-butane derivatives according to the general formula I in Claim 1 are mixed with inert, non-toxic, pharmaceutically suitable excipients.